# EUROPEAN PATENT APPLICATION

(11) **EP 4 851 599 A1**
(43) Date of publication of application: **09.12.2026**
(21) Application number: 25760980.0
(22) Date of filing: 25.02.2025
(51) Int. Cl.: G01N 1/00, A61M 1/02, G01N 1/10

(54) **COLLECTING KIT, AND COLLECTING METHOD**

(30) Priority: 29.02.2024 JP 2024029422
(71) Applicant: Terumo Kabushiki Kaisha, Tokyo 151-0072 (JP)
(72) Inventor: OKABE, Hiroshi, Fujinomiya-shi, Shizuoka 418-0004 (JP)
(74) Representative: TBK
(86) International application number: PCT/JP2025/006364
(87) International publication number: WO 2025/182899

(57) **Abstract**

A collection kit (10) includes an inflow tube (16) to which a medical bag (12) is connected, and a collection bag (18) that is connected to a downstream side of the inflow tube (16). The collection bag (18) includes a plurality of accommodation chambers (28) that accommodate a collection target (M), a single outflow port (36) for transferring the collection target (M) from a plurality of the accommodation chambers (28) to a plurality of the sampling containers (14), respectively, and a single main channel (30) that connects the inflow tube (16) and the outflow port (36). The collection bag (18) includes a plurality of connection channels (34) provided between the main channel (30) and a plurality of the accommodation chambers (28). The main channel (30) and each of a plurality of the accommodation chambers (28) are connected by a plurality of connection channels (34).

## Description

### Technical Field

The present invention relates to a collection kit and a collection method for transferring a collection target accommodated in a medical bag to a plurality of sampling containers.

### Background Art

Blood products include a red blood cell product, a plasma product, a platelet product, and a whole blood product. In order to ensure safety of the blood products, a small amount of sample may be collected to perform a culture test. In the culture test, a sample is collected into a culture bottle (sampling container), and the culture bottle is placed in an environment suitable for bacterial growth to detect the presence or absence of a pathogen.

Hereinafter, an operation procedure of transferring the platelet product from a platelet bag to two culture bottles will be described using a medical bag (hereinafter, referred to as a platelet bag) accommodating the platelet product as an example.

A small-capacity collection bag is connected to the platelet bag, and a part of the platelet product in the platelet bag is transferred to a collection bag. After that, the collection bag is disconnected from the platelet bag. Next, in a clean bench, the collection bag is connected to a tube extending from a sample collection pipe, and a prescribed amount of the platelet product is transferred from the collection bag to the sample collection pipe with reference to scales of the sample collection pipe. After that, two culture bottles are sequentially connected to the sample collection pipe, and a prescribed amount of the platelet product is transferred to the two culture bottles. One of the two culture bottles is a culture bottle used for anaerobic culture, and the other is a culture bottle used for aerobic culture.

For example, US Patent No. 8777921 discloses a collection kit for collecting a sample from a medical bag.

### Citation List

### Patent Literature

Patent Literature 1: US Patent No. 8777921

### Summary of Invention

From the viewpoint of further improving the safety of the blood product, culture tests for all blood products are being considered. Therefore, it is required to improve the work efficiency of the culture test for the blood product. Furthermore, in a case of performing the culture test on all blood products, it is desirable that a device for distributing the blood product into the culture bottles can be reduced in cost with a simple structure. Furthermore, it is desirable that a predetermined amount of a collection target can be collected into each of a plurality of sampling containers.

An object of the present invention is to solve the above-described problems.
(1) According to a first aspect of the present invention, there is provided a collection kit for collecting a collection target accommodated in a medical bag into a plurality of sampling containers, the collection kit including: an inflow tube to which the medical bag is connected; and a collection bag that is connected to a downstream side of the inflow tube and is formed by overlapping and welding two flexible sheets, in which the collection bag includes a plurality of accommodation chambers that accommodate the collection target, a single outflow port for transferring the collection target from a plurality of the accommodation chambers to a plurality of the sampling containers, respectively, at least one exhaust portion for exhausting air within a plurality of the accommodation chambers, a single main channel that connects the inflow tube and the outflow port, and a plurality of connection channels that are provided between the main channel and a plurality of the accommodation chambers and connects each of the accommodation chambers and the main channel.

In the collection kit, by providing the inflow tube to which the medical bag is connected, it is possible to improve work efficiency when the collection target is collected into a plurality of sampling containers. Furthermore, since the collection target can be accommodated in each of a plurality of the accommodation chambers, a predetermined amount of the collection target can be collected. By providing single outflow port for sequentially collecting the collection target into a plurality of the sampling containers, the collection kit including a plurality of the accommodation chambers can be reduced in cost with a simple structure.

(2) In the collection kit according to (1), the collection bag may include a connection portion to which a plurality of the connection channels and the main channel are connected, the main channel may have an inflow-side channel connecting the connection portion and the inflow tube, and an outflow-side channel connecting the connection portion and the outflow port, and the collection kit may include a channel switching portion that selectively connects any one of a plurality of the connection channels and the outflow-side channel.

With this configuration, the communication between any one of a plurality of the accommodation chambers and the outflow-side channel through the connection channel can be easily switched by the channel switching portion.

(3) In the collection kit according to (2), the collection bag may include a cutout portion obtained by cutting out a part of the flexible sheets in a thickness direction of the collection bag so as to surround the connection portion, and the channel switching portion may be provided in the cutout portion.

With this configuration, by providing the channel switching portion in the cutout portion, the channel switching portion can be mounted without crushing a plurality of the accommodation chambers and the main channel in the flexible sheet in the thickness direction.

(4) In the collection kit according to (2) or (3), the channel switching portion may include a switching member rotatable relative to the collection bag, the switching member may include a blocking portion that selectively blocks any one of a plurality of the connection channels, and one of a plurality of the connection channels may be blocked by the blocking portion by selecting a rotational position of the switching member.

With this configuration, by rotating the switching member, the communication state between one of a plurality of the connection channels and the outflow-side channel can be effectively switched.

(5) In the collection kit according to (4), the channel switching portion may include a housing that rotatably holds the switching member, the switching member may be provided so as to be movable in a thickness direction of the collection bag relative to the housing, and the switching member may move toward the connection channel in the thickness direction, thereby causing the blocking portion to press the connection channel toward the housing.

With this configuration, when the opened/blocked state of the connection channel is switched by the channel switching portion, the blocking portion can be pressed against the connection channel to effectively block the connection channel.

(6) In the collection kit according to (4) or (5), the blocking portion of the switching member may be capable of blocking the inflow-side channel, and one of a plurality of the connection channels and the inflow-side channel may be blocked by the blocking portion by selecting the rotational position of the switching member.

With this configuration, when one of a plurality of the connection channels is connected with the outflow-side channel, the movement of the collection target toward the inflow tube can be effectively prevented by blocking the communication of the inflow-side channel.

(7) In the collection kit according to (2) or (3), the channel switching portion may include a plurality of blocking portions capable of respectively blocking a plurality of the connection channels, a plurality of the blocking portions may include a plurality of blocking members respectively movable toward a plurality of the connection channels, and a plurality of the blocking members may be capable of selectively pressing a plurality of the connection channels.

With this configuration, by moving each of the blocking members of a plurality of the blocking portions, it is possible to selectively press and block a plurality of the connection channels.

(8) In the collection kit according to (7), the channel switching portion may include a single housing that movably holds a plurality of the blocking members, and a plurality of the blocking portions may include a plurality of pressing members respectively having a plurality of operation portions provided in the housing and exposed from the housing, the pressing members pushing the blocking members toward each of a plurality of the connection channels by operating the operation portions.

With this configuration, by operating each of a plurality of the operation portions, the blocking member can be effectively moved toward each of the plurality of connection channels by the pressing member. By providing a plurality of the blocking portions in the single housing, the channel switching portion can be compactly configured.

(9) According to a second aspect of the present invention, there is provided a collection method for collecting a collection target accommodated in a medical bag into a plurality of sampling containers via a collection kit, in which the collection kit includes an inflow tube to which the medical bag is connected, and a collection bag that is connected to a downstream side of the inflow tube and is formed by overlapping and welding two flexible sheets, the collection bag includes a plurality of accommodation chambers that accommodate the collection target, a single outflow port for transferring the collection target from a plurality of the accommodation chambers to a plurality of the sampling containers, respectively, at least one exhaust portion for exhausting air within a plurality of the accommodation chambers, a single main channel that connects the inflow tube and the outflow port, a plurality of connection channels that are provided between the main channel and a plurality of the accommodation chambers, and connect each of the accommodation chambers and the main channel, a connection portion that connects a plurality of the connection channels and the main channel, an inflow-side channel that is provided in the main channel and connects the connection portion and the inflow tube, and an outflow-side channel that is provided in the main channel and connects the connection portion and the outflow port, the collection method includes: a connection step of connecting the medical bag to the inflow tube; an introduction step of introducing the collection target having moved from the medical bag to an inside of the collection bag through the inflow tube and the inflow-side channel into each of a plurality of the accommodation chambers through a plurality of the connection channels; a separation step of separating the medical bag from the inflow tube after the collection target is introduced into each of a plurality of the accommodation chambers; and a collection step of sequentially transferring the collection target from a plurality of the accommodation chambers to a plurality of the sampling containers by sequentially connecting a plurality of the sampling containers to the outflow port and selectively blocking and opening a plurality of the connection channels.

In the collection method, by providing the inflow tube to which the medical bag is connected, it is possible to improve work efficiency when the collection target is collected into a plurality of sampling containers. Furthermore, since the collection target can be accommodated in each of a plurality of the accommodation chambers, a predetermined amount of the collection target can be collected. By providing the single outflow port for sequentially collecting the collection target into a plurality of the sampling containers, the collection kit including a plurality of the accommodation chambers can be reduced in cost with a simple structure.

According to the present invention, the collection bag includes the single main channel connecting the inflow tube and the outflow port, and a plurality of the connection channels provided between the main channel and a plurality of the accommodation chambers and connecting each of the accommodation chambers and the main channel, and includes the inflow tube to which the medical bag is connected. With this configuration, it is possible to improve work efficiency when the collection target is collected into a plurality of the sampling containers. Furthermore, since the collection target can be accommodated in each of a plurality of the accommodation chambers, a predetermined amount of the collection target can be collected. By providing the single outflow port for sequentially collecting the collection target into a plurality of the sampling containers, the collection kit including a plurality of the accommodation chambers can be reduced in cost with a simple structure.

### Brief Description of Drawings

[Fig. 1] Fig. 1 is a schematic front view of a collection kit according to a first embodiment of the present invention.
[Fig. 2] Fig. 2 is an explanatory view illustrating a case where a medical bag is connected to the collection kit of Fig. 1.
[Fig. 3] Fig. 3 is an enlarged plan view of a channel switching portion in a collection kit.
[Fig. 4] Fig. 4 is a cross-sectional view taken along line IV-IV of Fig. 3.
[Fig. 5] Fig. 5 is an explanatory view illustrating a case of transferring a collection target from a first accommodation chamber to a first sampling container.
[Fig. 6] Fig. 6 is an explanatory view illustrating a first position of a channel switching portion in Fig. 5.
[Fig. 7] Fig. 7 is an explanatory view illustrating a case of transferring a collection target from a second accommodation chamber to a second sampling container.
[Fig. 8] Fig. 8 is an explanatory view illustrating a second position of a channel switching portion in Fig. 7.
[Fig. 9] Fig. 9 is a cross-sectional view illustrating a state where a main channel is blocked by a channel switching portion.
[Fig. 10] Fig. 10 is a schematic front view of a collection kit according to a modification example.
[Fig. 11] Fig. 11 is a schematic front view of a collection kit according to a second embodiment of the present invention.
[Fig. 12] Fig. 12 is an enlarged plan view of a channel switching portion in a collection kit.
[Fig. 13] Fig. 13A is a cross-sectional view taken along line XIIIA-XIIIA in Fig. 12. Fig. 13B is a cross-sectional view taken along line XIIIB-XIIIB in Fig. 12.
[Fig. 14] Fig. 14 is an exploded perspective view of a channel switching portion and a collection bag.
[Fig. 15] Fig. 15 is a cross-sectional view taken along line XV-XV of Fig. 12.
[Fig. 16] Fig. 16 is a first explanatory view illustrating a switching state of a channel switching portion.
[Fig. 17] Fig. 17 is a cross-sectional view taken along line XVII-XVII of Fig. 16.
[Fig. 18] Fig. 18 is a second explanatory view illustrating a switching state of a channel switching portion.
[Fig. 19] Fig. 19 is a cross-sectional view taken along line XIX-XIX in Fig. 18.
[Fig. 20] Fig. 20 is a third explanatory view illustrating a switching state of a channel switching portion.

### Description of Embodiments

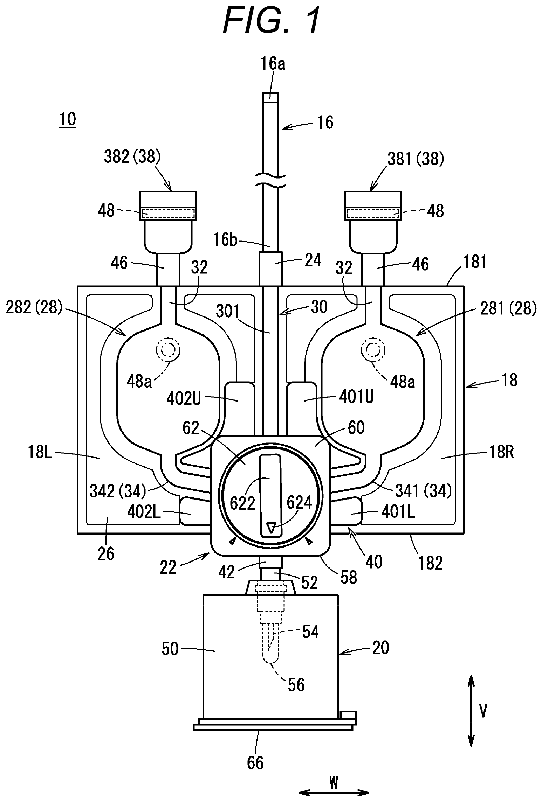

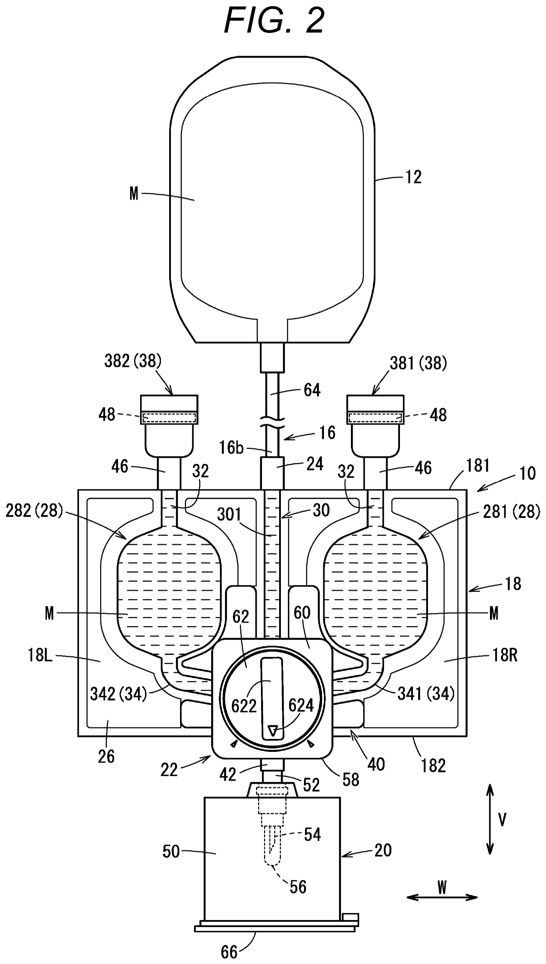

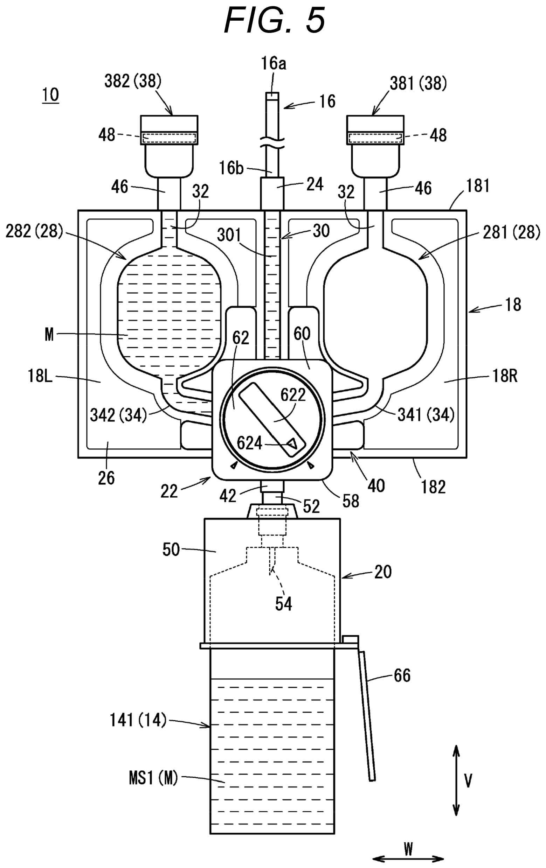

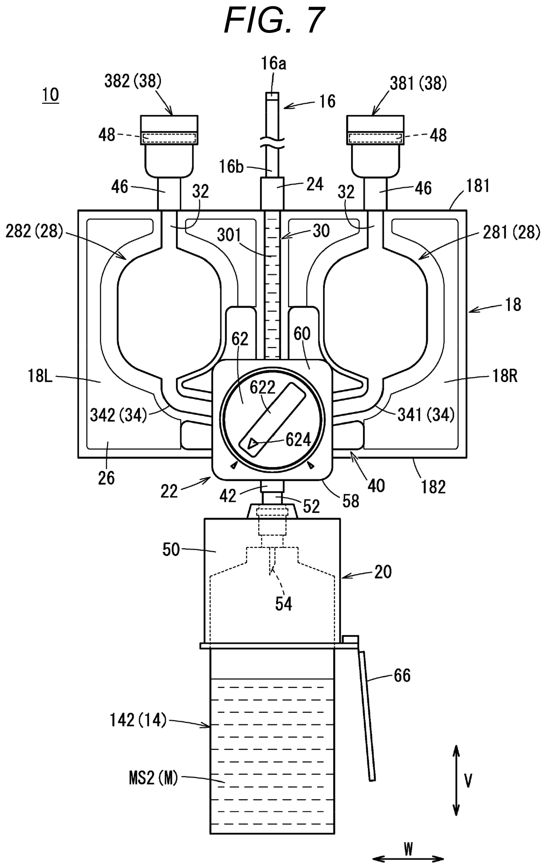

As illustrated in Fig. 1, a collection kit 10 according to a first embodiment is used to transfer a collection target M accommodated in a medical bag 12 illustrated in Fig. 2 to a plurality of sampling containers 14 (see Figs. 5 and 7). The collection target M is, for example, a blood product such as a platelet product. The collection target M may be a medication other than the blood product. The collection target M may be a liquid sample other than the medication. The medical bag 12 may be a blood bag system for centrifuging blood containing a plurality of components into a plurality of components having different specific gravities (for example, three components of a low-specific-gravity component, a medium-specific-gravity component, and a high-specific-gravity component, or two components of a low-specific-gravity component and a high-specific-gravity component), and separately accommodating and storing each of the components in different bags. In this case, the collection kit 10 may be connected to the blood bag system.

As illustrated in Fig. 1, the collection kit 10 includes an inflow tube 16 to which the medical bag 12 (see Fig. 2) is connected, a collection bag 18, an adapter 20, and a channel switching portion 22. The adapter 20 may be detachable from the collection bag 18. Hereinafter, an extending direction of the inflow tube 16 in the collection kit 10 illustrated in Fig. 1 (a direction of arrow V) is referred to as an up-down direction. A direction orthogonal to the up-down direction (a direction of arrow W) is referred to as a width direction of the collection kit 10, and a direction orthogonal to the up-down direction and the width direction is referred to as a thickness direction of the collection kit 10. The collection kit 10 is generally used in an orientation in which the up-down direction is aligned with a vertical direction.

The inflow tube 16 is a transparent or semitransparent medical tube. The inflow tube 16 is made of, for example, a thermoplastic resin such as a vinyl chloride resin. The inflow tube 16 can be connected to or separated from another medical tube without exposing the inside thereof to the outside air by using an aseptic connection device, a tube sealer, or the like.

The inflow tube 16 has an upstream end portion 16a and a downstream end portion 16b. In an initial state where the collection kit 10 is provided as a product, the upstream end portion 16a is sealed by welding. The downstream end portion 16b is connected to an upper side portion 181 of the collection bag 18 via a relay member 24. The relay member 24 is, for example, a sleeve made of a material harder than that of the collection bag 18.

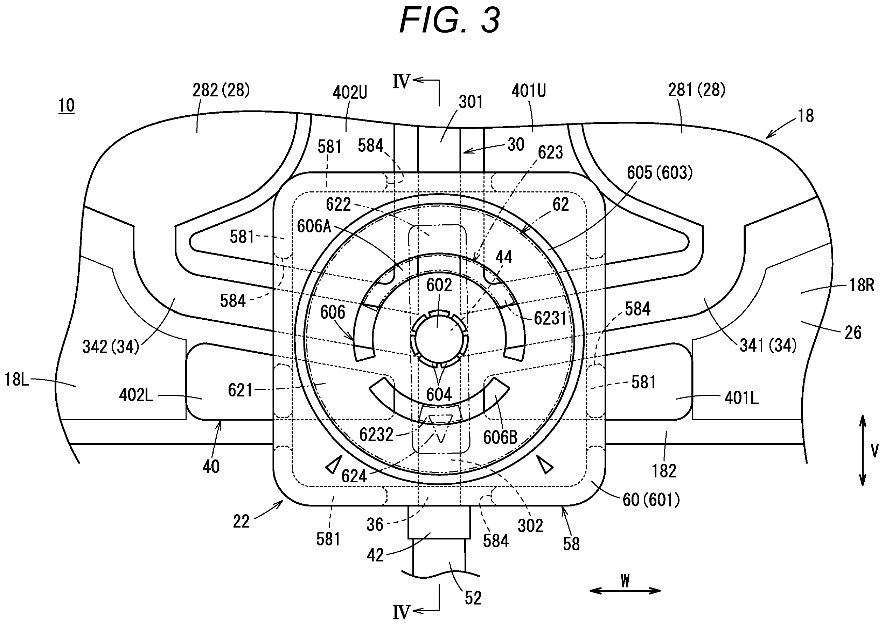

The collection bag 18 is connected to a downstream side of the inflow tube 16. The collection bag 18 includes two flexible sheets 26 that are overlapped in the thickness direction and welded to each other. The collection bag 18 includes a plurality of accommodation chambers 28, a single main channel 30, an exhaust channel 32, a plurality of connection channels 34, a single outflow port 36 (see Fig. 3), an exhaust portion 38, and a cutout portion 40. The main channel 30, the exhaust channel 32, the plurality of connection channels 34, and the plurality of accommodation chambers 28 are disposed between the two flexible sheets 26, and each of them bulges in the thickness direction of the collection bag 18.

The collection bag 18 includes a first region 18R and a second region 18L. The first region 18R is a region located on the right side from the center of the collection bag 18 in the width direction. The second region 18L is a region located on the left side from the center of the collection bag 18 in the width direction.

The plurality of accommodation chambers 28 accommodate the collection target M (see Fig. 2). The plurality of accommodation chambers 28 include a first accommodation chamber 281 and a second accommodation chamber 282. The first accommodation chamber 281 has an elliptical shape elongated in the up-down direction of the collection bag 18. The first accommodation chamber 281 is provided in the first region 18R. The volume of the first accommodation chamber 281 is, for example, about 8 mL to about 10 mL. The first accommodation chamber 281 temporarily stores the collection target M collected into a first sampling container 141 (see Fig. 5). Note that the plurality of accommodation chambers 28 are not limited to a case where two accommodation chambers 28 are provided. The plurality of accommodation chambers 28 may include three or more accommodation chambers 28.

The second accommodation chamber 282 has an elliptical shape elongated in the up-down direction of the collection bag 18. The second accommodation chamber 282 is provided in the second region 18L. The volume of the second accommodation chamber 282 is, for example, about 8 mL to about 10 mL. The volume of the second accommodation chamber 282 and the volume of the first accommodation chamber 281 are the same. The second accommodation chamber 282 temporarily stores the collection target M collected into a second sampling container 142 (see Fig. 7). Note that each of the first accommodation chamber 281 and the second accommodation chamber 282 is not limited to an elliptical shape. Each of the first accommodation chamber 281 and the second accommodation chamber 282 may have a shape other than the elliptical shape. Furthermore, the volume of the first accommodation chamber 281 and the volume of the second accommodation chamber 282 may be different.

The main channel 30 connects the inflow tube 16 and the outflow port 36. The main channel 30 is disposed at the center of the collection bag 18 in the width direction. The main channel 30 is disposed between the first region 18R and the second region 18L and extends in the up-down direction. The main channel 30 includes an inflow-side channel 301 and an outflow-side channel 302 (see Fig. 3). As illustrated in Fig. 3, the inflow-side channel 301 connects the inflow tube 16 and a connection portion 44 to be described later. As illustrated in Fig. 1, the upper end portion of the inflow-side channel 301 is provided at the upper end of the collection bag 18 and connected to the inflow tube 16 via the relay member 24. As illustrated in Fig. 3, the outflow-side channel 302 connects a connection portion 44 to be described later and the outflow port 36. The lower end portion of the outflow-side channel 302 is provided at the lower end of the collection bag 18 and is connected to the outflow port 36 via the connection portion 44. The inflow-side channel 301 and the outflow-side channel 302 are linearly provided in the up-down direction (a direction of arrow V) of the collection bag 18.

As illustrated in Fig. 1, the exhaust channel 32 is provided in each of the first region 18R and the second region 18L of the collection bag 18. The exhaust channel 32 is connected to each of the uppermost portions of the first accommodation chamber 281 and the second accommodation chamber 282. The upper end of the exhaust channel 32 is disposed at the upper side portion 181 of the collection bag 18 and connected to the exhaust portion 38.

The plurality of connection channels 34 are provided between the first accommodation chamber 281 and second accommodation chamber 282 and the main channel 30. The plurality of connection channels 34 connect each of the first accommodation chamber 281 and the second accommodation chamber 282 to the main channel 30. The plurality of connection channels 34 include a first connection channel 341 and a second connection channel 342. The first connection channel 341 is provided in the first region 18R of the collection bag 18. The first connection channel 341 extends in the width direction of the collection bag 18 and is connected to the main channel 30. The first connection channel 341 is connected to a lower portion of the first accommodation chamber 281. The main channel 30 and the first accommodation chamber 281 communicate with each other through the first connection channel 341. The first connection channel 341 is inclined upward from the main channel 30 toward the first accommodation chamber 281.

The second connection channel 342 is provided in the second region 18L of the collection bag 18. The second connection channel 342 extends in the width direction of the collection bag 18 and is connected to the main channel 30. The second connection channel 342 is connected to the lower portion of the second accommodation chamber 282. The main channel 30 and the second accommodation chamber 282 communicate with each other through the second connection channel 342. The second connection channel 342 is inclined upward from the main channel 30 toward the second accommodation chamber 282.

The collection bag 18 further includes the connection portion 44. The connection portion 44 connects the first connection channel 341 and the second connection channel 342 to the main channel 30. In plan view seen from the thickness direction of the collection bag 18, the connection portion 44 has a substantially cross shape. The inflow-side channel 301 is disposed above the connection portion 44, and the inflow-side channel 301 is disposed to extend upward relative to the connection portion 44. The outflow-side channel 302 is disposed below the connection portion 44, and the outflow-side channel 302 is disposed to extend downward relative to the connection portion 44. The first connection channel 341 extends from the connection portion 44 toward one side in the width direction. The second connection channel 342 extends from the connection portion 44 toward the other side in the width direction.

The outflow port 36 is provided in the outflow-side channel 302 of the main channel 30. The outflow port 36 is provided to respectively transfer the collection target M from the first accommodation chamber 281 and the second accommodation chamber 282 to the first sampling container 141 and the second sampling container 142 (see Figs. 5 and 7).

As illustrated in Fig. 1, the exhaust portion 38 is provided to exhaust air inside the first accommodation chamber 281 and the second accommodation chamber 282. The exhaust portion 38 includes a first exhaust portion 381 and a second exhaust portion 382. The first exhaust portion 381 is connected to the upper end portion of the exhaust channel 32 communicating with the first accommodation chamber 281 via a mounting member 46. The second exhaust portion 382 is connected to the upper end portion of the exhaust channel 32 communicating with the second accommodation chamber 282 via the mounting member 46. The mounting member 46 seals a space between the collection bag 18 and each of the first exhaust portion 381 and the second exhaust portion 382 in a liquid-tight and air-tight manner.

The first exhaust portion 381 and the second exhaust portion 382 each include a filter 48 that allows passage of gas and blocks passage of liquid. In a case where the main component of the collection target M is water, the filter 48 is preferably a hydrophilic filter. In this case, when the collection target M moves to the filter 48 through the exhaust channel 32 and comes into contact with the filter 48, the pores of the filter 48 are blocked by the liquid. After the pores of the filter 48 are blocked, the filter 48 prevents passage of gas and liquid. Therefore, leakage of the collection target M from the first accommodation chamber 281 and the second accommodation chamber 282 to the outside is prevented. Note that an example of the collection target M whose main component is water is a blood product.

Note that the exhaust portion 38 is not limited to being connected to the upper end portion of the exhaust channel 32 via the mounting member 46. For example, a filter 48a may be provided at the flexible sheet 26 of the collection bag 18 (in Fig. 1, see two-dot chain line shape). In this case, the filter 48a is disposed on the wall portion of the flexible sheet 26 facing the first accommodation chamber 281 and the second accommodation chamber 282, and the air in the first accommodation chamber 281 and the second accommodation chamber 282 can be directly discharged to the atmosphere through the filter 48a.

As illustrated in Fig. 3, the cutout portion 40 is formed by cutting out a part of the flexible sheet 26 in the thickness direction of the collection bag 18. The cutout portion 40 is disposed so as to surround the connection portion 44. That is, the cutout portion 40 is provided at the center of the collection bag 18 in the width direction and from the center to the lower portion in the up-down direction. The cutout portion 40 includes a first upper hole portion 401U and a second upper hole portion 402U, and a first lower hole portion 401L and a second lower hole portion 402L. The first upper hole portion 401U and the first lower hole portion 401L are provided in the first region 18R. The first upper hole portion 401U is surrounded by the first accommodation chamber 281, the inflow-side channel 301 of the main channel 30, and the first connection channel 341. The first lower hole portion 401L is disposed below the first upper hole portion 401U with the first connection channel 341 interposed therebetween. The first lower hole portion 401L is surrounded by the outflow-side channel 302 of the main channel 30, the first connection channel 341, and the lower side portion 182 of the collection bag 18.

The second upper hole portion 402U and the second lower hole portion 402L are provided in the second region 18L. The second upper hole portion 402U is surrounded by the second accommodation chamber 282, the inflow-side channel 301 of the main channel 30, and the second connection channel 342. The second lower hole portion 402L is disposed below the second upper hole portion 402U with the second connection channel 342 interposed therebetween. The second lower hole portion 402L is surrounded by the outflow-side channel 302 of the main channel 30, the second connection channel 342, and the lower side portion 182 of the collection bag 18.

As illustrated in Fig. 1, the adapter 20 is connected to the lower end portion of the outflow-side channel 302 of the main channel 30 via a connection member 42. The adapter 20 is disposed so as to protrude downward relative to the lower side portion 182 of the collection bag 18. The adapter 20 includes an adapter body 50 and a communication tube portion 52 provided at the upper end portion of the adapter body 50. When the communication tube portion 52 is inserted into the inner hole of the connection member 42, the adapter 20 is connected to the main channel 30. The connection member 42 seals a space between the communication tube portion 52 and the collection bag 18 in a liquid-tight and air-tight manner.

The adapter 20 includes a needle tube 54 and a rubber cover 56 covering the needle tube 54. The needle tube 54 is provided inside the adapter body 50 and is connected to the communication tube portion 52. When the neck portion of the sampling container 14 is inserted into the adapter body 50, the needle tube 54 penetrates a stopper (not illustrated) of the sampling container 14 (see Figs. 5 and 7). As the connection member 42 is opened, the sampling container 14 and the outflow-side channel 302 of the main channel 30 communicate with each other via the communication tube portion 52 and the needle tube 54.

When the needle tube 54 punctures the stopper of the sampling container 14, the rubber cover 56 is compressed by being pushed toward the collection bag 18. The needle tube 54 penetrates the compressed rubber cover 56. When the needle tube 54 comes out of the stopper of the sampling container 14, the rubber cover 56 extends by an elastic restoring force to cover the needle tube 54 again.

The channel switching portion 22 selectively connects any one of the first connection channel 341 and the second connection channel 342 with the outflow-side channel 302 of the main channel 30. As illustrated in Fig. 3, the channel switching portion 22 is provided in the cutout portion 40 of the collection bag 18 and faces the connection portion 44. The channel switching portion 22 includes a housing 58, a cover member 60, and a switching member 62.

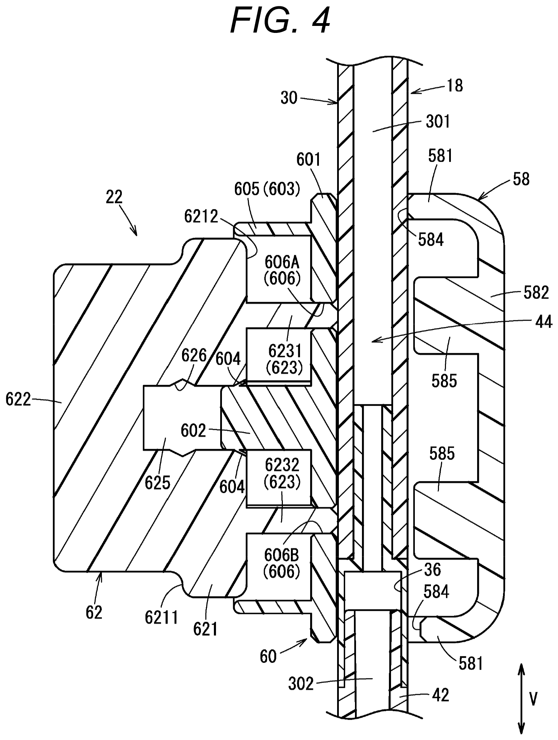

The housing 58 rotatably holds the switching member 62. In plan view of the channel switching portion 22, the housing 58 is formed in a rectangular shape. As illustrated in Fig. 4, the housing 58 has side walls 581 disposed on four sides and a bottom wall 582. Each of the side walls 581 has a recessed portion 584. Each recessed portion 584 is recessed from one end of the side wall 581 toward the bottom wall 582. The bottom wall 582 is surrounded by the other end of each side wall 581. The bottom wall 582 has a pedestal 585. The pedestal 585 protrudes annularly from the bottom wall 582.

As illustrated in Fig. 3, a part of the collection bag 18 is accommodated inside the housing 58. The connection portion 44 of the collection bag 18 is accommodated inside the housing 58. The side wall 581 of the housing 58 is disposed below the cutout portion 40 and the lower side portion 182 of the collection bag 18 (see Fig. 3). The connection portion 44 of the collection bag 18 is accommodated in a center portion of the housing 58. The inflow-side channel 301 and the outflow-side channel 302 of the main channel 30, and the first connection channel 341 and the second connection channel 342 are disposed in each recessed portion 584. A part of the side wall 581 of the housing 58 is disposed in each of the first upper hole portion 401U and the second upper hole portion 402U, the first lower hole portion 401L, and the second lower hole portion 402L of the cutout portion 40. The cutout portion 40 prevents contact between the side wall 581 of the housing 58 and the collection bag 18.

As illustrated in Fig. 4, the cover member 60 is mounted on the side walls 581 of the housing 58 to close the opening. The connection portion 44 of the collection bag 18 is accommodated between the cover member 60 and the housing 58. The cover member 60 includes a lid portion 601, a support shaft 602, and a guide portion 603. As illustrated in Fig. 3, in plan view of the cover member 60, the lid portion 601 has a rectangular shape. As illustrated in Fig. 4, the lid portion 601 is formed in a plate shape. The support shaft 602 is provided at the center of the lid portion 601 and protrudes from the lid portion 601. The axial direction of the support shaft 602 and the lid portion 601 are substantially perpendicular to each other. The outer peripheral surface of the support shaft 602 has a plurality of protrusions 604. The plurality of protrusions 604 are provided at the distal portion of the support shaft 602. The plurality of protrusions 604 are disposed along the circumferential direction on the outer peripheral surface of the support shaft 602 (see Fig. 3). The plurality of protrusions 604 protrude radially outward from the outer peripheral surface of the support shaft 602. The plurality of protrusions 604 are formed to be tapered toward the distal end of the support shaft 602.

The guide portion 603 includes an annular wall 605 and a guide groove 606. The annular wall 605 has an annular shape centered on the support shaft 602. The annular wall 605 protrudes from the lid portion 601 along the axial direction of the support shaft 602. The guide groove 606 penetrates the lid portion 601 inside the annular wall 605. As illustrated in Fig. 3, the guide groove 606 includes a first groove portion 606A and a second groove portion 606B. The first groove portion 606A and the second groove portion 606B have an arc shape having a radius centered on the support shaft 602. In the circumferential direction about the support shaft 602, the first groove portion 606A and the second groove portion 606B are spaced apart from each other. The length of the first groove portion 606A in the circumferential direction is longer than the length of the second groove portion 606B in the circumferential direction.

The switching member 62 is provided to be rotatable relative to the collection bag 18. The switching member 62 is provided to be rotatable within the annular wall 605 of the cover member 60. As illustrated in Fig. 4, the switching member 62 is provided to be movable in the thickness direction of the collection bag 18 relative to the housing 58. The switching member 62 includes a main body portion 621, an operation portion 622, and a blocking portion 623.

The main body portion 621 has a disk shape (see Fig. 3). The main body portion 621 is accommodated within the annular wall 605. An insertion hole 625 into which the support shaft 602 is inserted is provided at the center of the main body portion 621. The operation portion 622 is gripped by a user when the user rotates the switching member 62 of the channel switching portion 22. The operation portion 622 protrudes in the axial direction from a first surface 6211 of the main body portion 621. As illustrated in Fig. 3, the operation portion 622 linearly extends in the radial direction of the main body portion 621 so as to pass through the center of the main body portion 621. Both ends of the operation portion 622 extend to the vicinity of the outer peripheral surface of the main body portion 621. At one end portion of the operation portion 622, for example, a mark 624 recognizable by the user is provided (see Fig. 1). As illustrated in Fig. 4, the insertion hole 625 of the main body portion 621 extends from the main body portion 621 to a part of operation portion 622. The support shaft 602 of the cover member 60 is inserted into the insertion hole 625. The switching member 62 is rotatably supported relative to the cover member 60. The main body portion 621 is provided to be movable along the axial direction of the support shaft 602. The axial direction of the support shaft 602 is the thickness direction of the collection bag 18 when the channel switching portion 22 is mounted on the collection bag 18.

The insertion hole 625 has an engagement recess 626. The engagement recess 626 is expanded radially outward such that each of the protrusions 604 of the support shaft 602 is engageable therewith. The engagement recess 626 has an annular shape extending in the circumferential direction of the insertion hole 625.

As illustrated in Fig. 3, the blocking portion 623 is provided so as to be able to selectively block any one of the first connection channel 341 and the second connection channel 342. As illustrated in Fig. 4, the blocking portion 623 is provided on a second surface 6212 opposite to the first surface 6211 of the main body portion 621. The blocking portion 623 includes a first blocking piece 6231 and a second blocking piece 6232.

The first blocking piece 6231 and the second blocking piece 6232 protrude from the second surface 6212 in the axial direction. As illustrated in Fig. 3, each of the first blocking piece 6231 and the second blocking piece 6232 has an arc shape having a radius centered on the axis of the main body portion 621. In the circumferential direction of the switching member 62, the first blocking piece 6231 and the second blocking piece 6232 are spaced apart from each other. The length of the first blocking piece 6231 in the circumferential direction is longer than the length of the second blocking piece 6232 in the circumferential direction. In plan view of the switching member 62, the center of the first blocking piece 6231 in the length direction and the other end portion of the operation portion 622 are disposed at the same position. The second blocking piece 6232 and one end portion of the operation portion 622 are disposed at the same position. The first blocking piece 6231 is inserted into the first groove portion 606A. The second blocking piece 6232 is inserted into the second groove portion 606B. In the circumferential direction of the switching member 62, the length of the second groove portion 606B is longer than the length of the second blocking piece 6232. When the switching member 62 rotates relative to the cover member 60, the first blocking piece 6231 is movable along the first groove portion 606A, and the second blocking piece 6232 is movable along the second groove portion 606B. As illustrated in Fig. 4, the first blocking piece 6231 and the second blocking piece 6232 face the pedestal 585 of the housing 58.

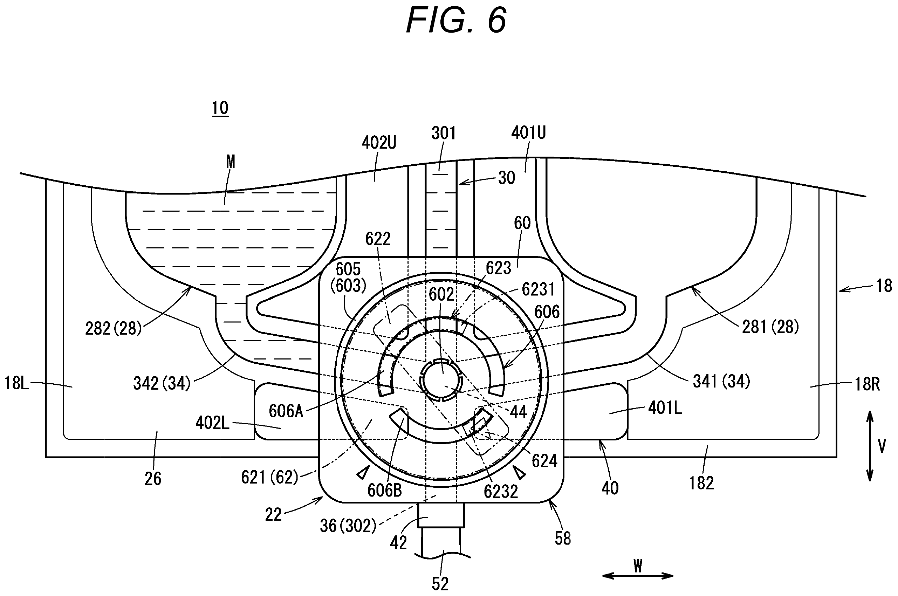

By selecting the rotational position of the switching member 62, one connection channel 34, which is either the first connection channel 341 or the second connection channel 342, can be blocked by the first blocking piece 6231 of the blocking portion 623. Specifically, in plan view of the switching member 62 illustrated in Fig. 5, when the switching member 62 is rotated in a first direction from the initial position, as illustrated in Fig. 6, the first position of the channel switching portion 22 is reached, and the first blocking piece 6231 faces the inflow-side channel 301 of the main channel 30 and the second connection channel 342. The second blocking piece 6232 is disposed in the cutout portion 40 (first lower hole portion 401L) between the first connection channel 341 and the outflow-side channel 302.

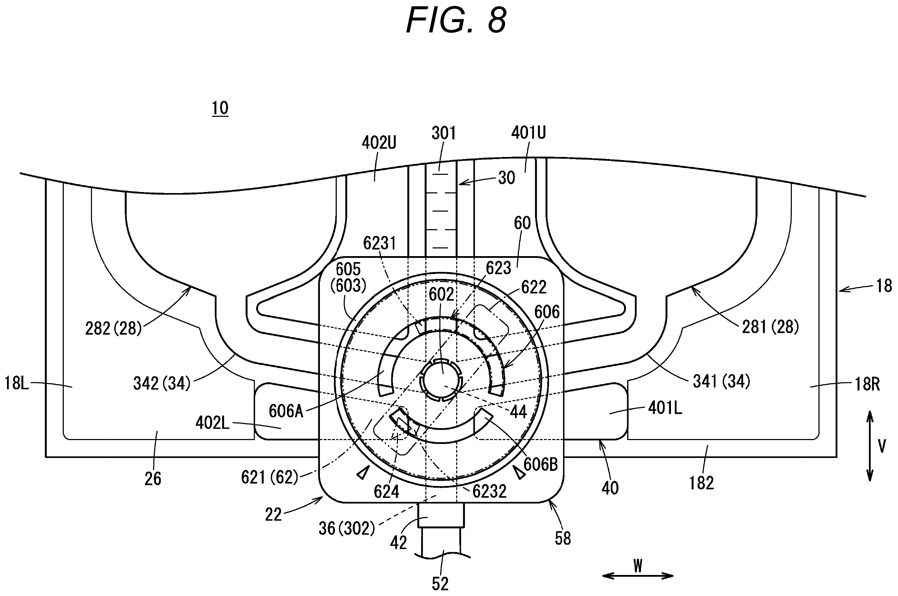

In plan view of the switching member 62 illustrated in Fig. 7, when the switching member 62 is rotated from the initial position in a second direction, which is the direction opposite to the first direction, as illustrated in Fig. 8, the second position of the channel switching portion 22 is reached, and the first blocking piece 6231 faces the inflow-side channel 301 of the main channel 30 and the first connection channel 341. The second blocking piece 6232 is disposed in the cutout portion 40 (second lower hole portion 402L) between the second connection channel 342 and the outflow-side channel 302.

The switching member 62 is rotatably supported via the support shaft 602 and is supported movably in the axial direction via the support shaft 602. As illustrated in Fig. 4, at an open position where the blocking portion 623 of the switching member 62 and the pedestal 585 of the housing 58 are spaced apart from each other, the first connection channel 341 and the second connection channel 342, the main channel 30, and the blocking portion 623 are spaced apart and in an open communication state.

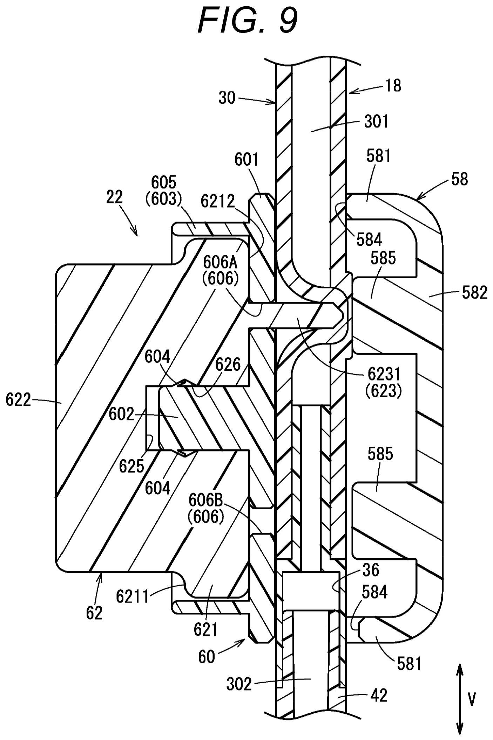

As illustrated in Fig. 9, when the user pushes the switching member 62 toward the pedestal 585 in the thickness direction of the collection bag 18, the switching member 62 moves along the support shaft 602 toward the pedestal 585 of the housing 58 to the blocking position. At the blocking position, the blocking portion 623 approaches the pedestal 585, the main channel 30 is clamped between the blocking portion 623 and the pedestal 585, such that the main channel 30 reaches a blocked state.

At the initial position of the channel switching portion 22 illustrated in Fig. 3, the inflow-side channel 301 of the main channel 30 is blocked by the movement of the switching member 62 toward the pedestal 585. At the first position of the channel switching portion 22 illustrated in Fig. 6, the second connection channel 342 and the inflow-side channel 301 are blocked by the movement of the switching member 62 toward the pedestal 585. At the second position of the channel switching portion 22 illustrated in Fig. 8, the first connection channel 341 and the inflow-side channel 301 are blocked by the movement of the switching member 62 toward the pedestal 585.

Note that in another embodiment of the collection kit 10, at the first position of the channel switching portion 22, the first blocking piece 6231 may block only the second connection channel 342 with the first blocking piece 6231. At the second position of the channel switching portion 22, only the first connection channel 341 may be blocked by the first blocking piece 6231.

Next, a collection method for dispensing the collection target M into the first sampling container 141 and the second sampling container 142 using the collection kit 10 according to the first embodiment will be described.

First, as illustrated in Fig. 2, a connection step of connecting the medical bag 12 to the inflow tube 16 is performed. The user connects the medical bag 12 accommodating the collection target M such as a platelet product to the inflow tube 16. At this time, the medical bag 12 is disposed above the collection bag 18. The user joins a supply tube 64 of the medical bag 12 to the inflow tube 16.

Next, an introduction step of introducing the collection target M that has moved from the medical bag 12 to the inside of the collection bag 18 through the inflow tube 16 and the inflow-side channel 301 into the first accommodation chamber 281 and the second accommodation chamber 282 through the first connection channel 341 and the second connection channel 342 is performed. Before moving the collection target M to the first accommodation chamber 281, the user sets the switching member 62 of the channel switching portion 22 to the initial position as illustrated in Fig. 3. The initial position is a position where the switching member 62 is disposed so as to be spaced apart from the housing 58 in the thickness direction of the collection bag 18, the first blocking piece 6231 faces the inflow-side channel 301 of the main channel 30, and the second blocking piece 6232 faces the outflow-side channel 302 of the main channel 30. As illustrated in Fig. 4, at the initial position, the first blocking piece 6231 and the second blocking piece 6232 are spaced apart from the main channel 30 in the thickness direction of the collection bag 18, and the communication of the main channel 30 is not blocked, resulting in the open position. Therefore, the inflow-side channel 301, the first connection channel 341, and the second connection channel 342 communicate with each other via the connection portion 44.

The collection target M is, for example, a liquid and is flowable. Therefore, as illustrated in Fig. 2, the collection target M flows out of the medical bag 12 by gravity and flows into the inflow-side channel 301 of the main channel 30 via the supply tube 64 and the inflow tube 16. The collection target M moves downward from the inflow tube 16 along the inflow-side channel 301 by gravity. The collection target M branches into the first connection channel 341 and the second connection channel 342 at the connection portion 44, and flows into the first accommodation chamber 281 and the second accommodation chamber 282 through the first connection channel 341 and the second connection channel 342.

When the collection target M flows into the first accommodation chamber 281, the air remaining in the first accommodation chamber 281 is discharged from the exhaust channel 32 to the atmosphere through the first exhaust portion 381. When the collection target M flows into the second accommodation chamber 282, the air remaining in the second accommodation chamber 282 is discharged from the exhaust channel 32 to the atmosphere through the second exhaust portion 382.

After the first accommodation chamber 281 and the second accommodation chamber 282 are filled with the collection target M, the collection target M flows into the filter 48 of each of the first exhaust portion 381 and the second exhaust portion 382 via the exhaust channel 32. When the collection target M comes into contact with the filter 48, the collection target M is captured and held in the pores of the filter 48. Thus, the filter 48 blocks the collection target M in the first exhaust portion 381 and the second exhaust portion 382. Therefore, the first exhaust portion 381 and the second exhaust portion 382 become clogged, and thus the collection target M is prevented from passing through the filter 48 and being discharged to the outside. The necessary amount (for example, about 8 mL to about 10 mL) of the collection target M is accommodated in each of the first accommodation chamber 281 and the second accommodation chamber 282. The supply of the collection target M from the medical bag 12 to the collection bag 18 is stopped.

Next, a separation step of separating the medical bag 12 from the inflow tube 16 is performed. The inflow tube 16 is disconnected from the supply tube 64 of the medical bag 12, and at the same time, the upstream end portion 16a of the inflow tube 16 is sealed by welding. For welding the inflow tube 16, for example, a tube sealer such as a high-frequency sealer or an ultrasonic sealer is used. The medical bag 12 disconnected from the collection kit 10 is stored until the test is completed.

Next, a collection step of sequentially transferring the collection target M from the first accommodation chamber 281 and the second accommodation chamber 282 to the first sampling container 141 and the second sampling container 142 by sequentially connecting the first sampling container 141 and the second sampling container 142 to the outflow port 36 and selectively blocking and opening the first connection channel 341 and the second connection channel 342 is performed. Hereinafter, a case where the collection target M in the first accommodation chamber 281 is transferred to the first sampling container 141 and then the collection target M in the second accommodation chamber 282 is transferred to the second sampling container 142 will be described.

Note that the transfer of the collection target M is not limited to a case where the collection target M is sequentially transferred to the first sampling container 141 and the second sampling container 142. For example, after the collection target M in the second accommodation chamber 282 is transferred to the second sampling container 142, the collection target M in the first accommodation chamber 281 may be transferred to the first sampling container 141.

First, a step of transferring the collection target M in the first accommodation chamber 281 to the first sampling container 141 is performed. The user grips the operation portion 622 of the switching member 62 and rotates the switching member 62 from the initial position to the first position (see Fig. 5). As illustrated in Fig. 6, at the first position of the switching member 62, the first blocking piece 6231 faces the second connection channel 342 and the inflow-side channel 301, and the second blocking piece 6232 is disposed between the first connection channel 341 and the outflow-side channel 302. After the switching member 62 is rotated to the first position, the user pushes the switching member 62 toward the housing 58 to reach the blocking position.

At the blocking position, the switching member 62 moves in the axial direction toward the housing 58 along the support shaft 602. The main body portion 621 of the switching member 62 is accommodated within the annular wall 605 of the guide portion 603 (see Fig. 9). The first blocking piece 6231 moves toward the main channel 30 and the second connection channel 342, and presses the main channel 30 and the second connection channel 342 toward the pedestal 585. The main channel 30 and the second connection channel 342 are deformed by being crushed between the first blocking piece 6231 and the pedestal 585, and the inflow-side channel 301 of the main channel 30 and the second connection channel 342 are blocked. As illustrated in Fig. 9, after the switching member 62 moves along the support shaft 602, the protrusion 604 of the support shaft 602 is engaged with the engagement recess 626, whereby the movement of the switching member 62 in the axial direction relative to the support shaft 602 is prevented. The switching member 62 is locked at the blocking position where the switching member 62 has moved toward the housing 58. At the blocking position, the first connection channel 341 and the outflow-side channel 302 of the main channel 30 communicate with each other through the connection portion 44. The first accommodation chamber 281 and the outflow-side channel 302 communicate with each other via the first connection channel 341.

As illustrated in Fig. 5, the first sampling container 141 is connected to the adapter 20. The lid 66 of the adapter 20 is opened, and the neck portion of the first sampling container 141 is inserted into the adapter body 50. Thus, the needle tube 54 provided within the adapter body 50 penetrates the stopper of the first sampling container 141.

The user bends the plug of the connection member 42 to break the plug, thereby opening the outflow-side channel 302 of the main channel 30. The collection target M in the first accommodation chamber 281 is sucked out by the negative pressure inside the first sampling container 141, passes through the first connection channel 341, the outflow-side channel 302, the outflow port 36, the connection member 42, the communication tube portion 52 of the adapter 20, and the needle tube 54, and flows into the first sampling container 141. At this time, since the communication of the second connection channel 342 and the outflow-side channel 302 is blocked by the channel switching portion 22, the collection target M in the second accommodation chamber 282 is not sucked out through the outflow-side channel 302. Thus, substantially the entire amount of the collection target M stored in the first accommodation chamber 281 is transferred to the first sampling container 141. The amount of the collection target M in the first sampling container 141 is, for example, about 8 mL to about 10 mL.

After the first sampling container 141 is removed from the adapter 20, as illustrated in Fig. 7, a step of connecting the second sampling container 142 to the adapter 20 and transferring the collection target M in the second accommodation chamber 282 to the second sampling container 142 is performed. Since the connection of the second sampling container 142 is the same as the connection of the first sampling container 141, a detailed description of the connection between the adapter 20 and the second sampling container 142 will be omitted.

After the second sampling container 142 is connected, as illustrated in Fig. 8, the switching member 62 of the channel switching portion 22 is rotated to reach the second position. The second position is switched by rotating the switching member 62 in a direction opposite to that in the case of switching from the initial position to the first position. At the second position of the channel switching portion 22, the first blocking piece 6231 faces the first connection channel 341 and the inflow-side channel 301, and the second blocking piece 6232 is disposed between the second connection channel 342 and the outflow-side channel 302. At the second position, the switching member 62 remains at the blocking position where the switching member 62 has been pushed toward the housing 58 (see Fig. 9).

By setting the switching member 62 to the second position, the main channel 30 and the first connection channel 341 are pressed toward the pedestal 585 by the first blocking piece 6231. The main channel 30 and the first connection channel 341 are crushed between the first blocking piece 6231 and the pedestal 585, and the inflow-side channel 301 of the main channel 30 and the first connection channel 341 are blocked. On the other hand, the second connection channel 342 is opened, and the second connection channel 342 and the outflow-side channel 302 of the main channel 30 communicate with each other through the connection portion 44.

By setting the switching member 62 of the channel switching portion 22 to the second position, the collection target M in the second accommodation chamber 282 is sucked out by the negative pressure in the second sampling container 142. The collection target M passes through the second connection channel 342, the outflow-side channel 302, the outflow port 36, the connection member 42, and the communication tube portion 52 and the needle tube 54 of the adapter 20 from the second accommodation chamber 282, and flows into the second sampling container 142. Thus, substantially the entire amount of the collection target M stored in the second accommodation chamber 282 is transferred to the second sampling container 142. The amount of the collection target M in the second sampling container 142 is, for example, about 8 mL to about 10 mL. The second sampling container 142 is removed from the adapter 20. Thus, the collection (sampling) of the collection target M using the collection kit 10 is completed. That is, a first sample MS1 is introduced into the first sampling container 141, and a second sample MS2 is introduced into the second sampling container 142.

In a case where the collection target M is a blood product, a culture test is performed for the first sample MS1 and the second sample MS2. Specifically, the first sample MS1 in the first sampling container 141 is used, for example, for anaerobic culture, and whether or not anaerobic bacteria grow is observed. The second sample MS2 in the second sampling container 142 is used, for example, for aerobic culture, and whether or not aerobic bacteria grow is observed. In both tests, the first sampling container 141 and the second sampling container 142 are used as culture bottles. In a case where the first sample MS1 and the second sample MS2 pass the culture test, the collection target M in the medical bag 12 from which the first sample MS1 and the second sample MS2 are collected is determined to be a passed product. The passed product is used for patient treatment (blood transfusion and the like).

Note that in a case where the collection target M is dispensed into the first sampling container 141 and the second sampling container 142 using the collection kit 10, the dispensing may be performed using a collection kit jig (not illustrated).

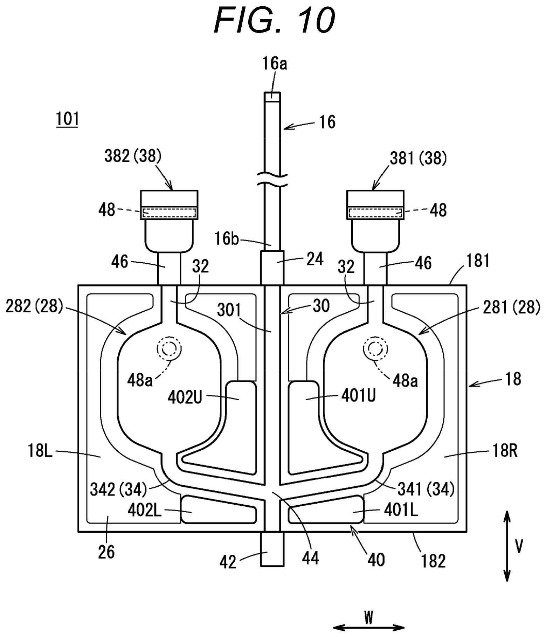

The collection kit 10 is not limited to the configuration including the channel switching portion 22. For example, a collection kit 101 according to a modification example illustrated in Fig. 10 does not include the channel switching portion 22 in the connection portion 44 that is a connection portion between the first connection channel 341 and second connection channel 342 and the main channel 30. For example, a blocking member (not illustrated) such as a clamp may be provided in each of the first connection channel 341 and the second connection channel 342, and the outflow-side channel 302 of the main channel 30 and any one of the first connection channel 341 and the second connection channel 342 may be selectively communicated by appropriately opening and closing two blocking members.

The first embodiment has the following effects.

As illustrated in Fig. 1, the collection bag 18 includes the single main channel 30 that connects the inflow tube 16 and the outflow port 36, and the plurality of connection channels 34 (the first connection channel 341 and the second connection channel 342) provided between the main channel 30 and the plurality of accommodation chambers 28 (the first accommodation chamber 281 and the second accommodation chamber 282) and connecting each of the accommodation chambers 28 and the main channel 30. By providing the inflow tube 16 to which the medical bag 12 is connected, it is possible to improve work efficiency when the collection target M is collected into the plurality of sampling containers 14 (see Figs. 5 and 7). Furthermore, since the collection target M can be accommodated in each of the plurality of accommodation chambers 28, a predetermined amount of the collection target M can be collected. By providing single outflow port 36 for sequentially collecting into the plurality of sampling containers 14, the collection kit 10 including the plurality of accommodation chambers 28 can be reduced in cost with a simple structure.

As illustrated in Fig. 3, the collection bag 18 has a connection portion 44 in which the plurality of connection channels 34 and the main channel 30 are connected, and the main channel 30 has the inflow-side channel 301 that connects the connection portion 44 and the inflow tube 16, and the outflow-side channel 302 that connects the connection portion 44 and the outflow port 36. The collection kit 10 includes the channel switching portion 22 that selectively connects any one of the plurality of connection channels 34 with the outflow-side channel 302.

Thus, the communication between any one of the plurality of accommodation chambers 28 and the outflow-side channel 302 through the connection channel 34 can be easily switched by the channel switching portion 22.

The collection bag 18 includes the cutout portion 40 obtained by cutting out a part of the flexible sheet 26 in the thickness direction of the collection bag 18 so as to surround the connection portion 44, and the channel switching portion 22 is provided in the cutout portion 40.

Thus, by disposing the channel switching portion 22 in the cutout portion 40, the channel switching portion 22 can be mounted without crushing the plurality of accommodation chambers 28 and the main channel 30 in the flexible sheet 26 in the thickness direction.

The channel switching portion 22 includes the switching member 62 rotatable relative to the collection bag 18, and the switching member 62 includes the blocking portion 623 that selectively blocks any one of the plurality of connection channels 34. By selecting the rotational position of the switching member 62, one of the plurality of connection channels 34 can be blocked by the blocking portion 623. Thus, by rotating the switching member 62, the communication state between any one of the plurality of connection channels 34 and the outflow-side channel 302 can be effectively switched.

As illustrated in Fig. 4, the channel switching portion 22 includes the housing 58 that rotatably holds the switching member 62, and the switching member 62 is provided to be movable in the thickness direction of the collection bag 18 relative to the housing 58. When the switching member 62 moves toward the connection channels 34 in the thickness direction of the collection bag 18, the connection channels 34 are pressed toward the housing 58 by the blocking portion 623. Thus, when the opened/blocked state of the connection channel 34 is switched by the channel switching portion 22, the blocking portion 623 can be pressed against the connection channel 34 to effectively block the connection channel 34.

As illustrated in Fig. 9, the blocking portion 623 of the switching member 62 can block the inflow-side channel 301. By selecting the rotational position of the switching member 62, one of the plurality of connection channels 34 and the inflow-side channel 301 can be blocked by the blocking portion 623. Thus, when one of the plurality of connection channels 34 is connected with the outflow-side channel 302, the movement of the collection target M toward the inflow tube 16 can be effectively prevented by blocking the communication of the inflow-side channel 301.

As illustrated in Figs. 5 and 7, the collection method for collecting the collection target M into the plurality of sampling containers 14 via the collection kit 10 includes a collection step of sequentially transferring the collection target M from the plurality of accommodation chambers 28 to the plurality of sampling containers 14 by sequentially connecting the plurality of sampling containers 14 to the outflow port 36 and selectively blocking and opening the plurality of connection channels 34 after performing a separation step of introducing the collection target M into each of the plurality of accommodation chambers 28 and separating the medical bag 12 from the inflow tube 16.

According to this collection method, the work efficiency when collecting the collection target M into the plurality of sampling containers 14 can be improved. Furthermore, since the collection target M can be accommodated in each of the plurality of accommodation chambers 28, a predetermined amount of the collection target M can be collected. By providing single outflow port 36 for sequentially collecting into the plurality of sampling containers 14, the collection kit 10 including the plurality of accommodation chambers 28 can be reduced in cost with a simple structure.

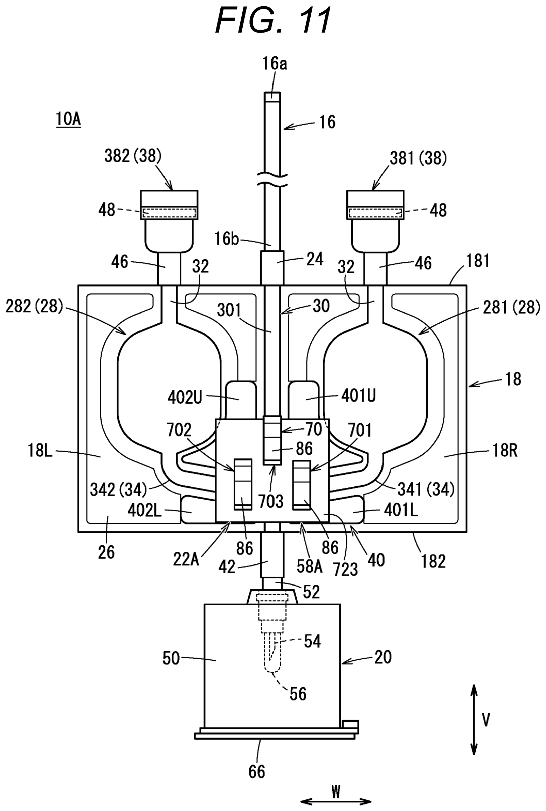

As illustrated in Fig. 11, a collection kit 10A according to a second embodiment includes a channel switching portion 22A that selectively connects one of the first connection channel 341 and the second connection channel 342 in the collection bag 18 with the outflow-side channel 302 of the main channel 30. Note that the same components as those of the collection kit 10 according to the first embodiment illustrated in Fig. 1 are denoted by the same reference signs, and the detailed description thereof will be omitted.

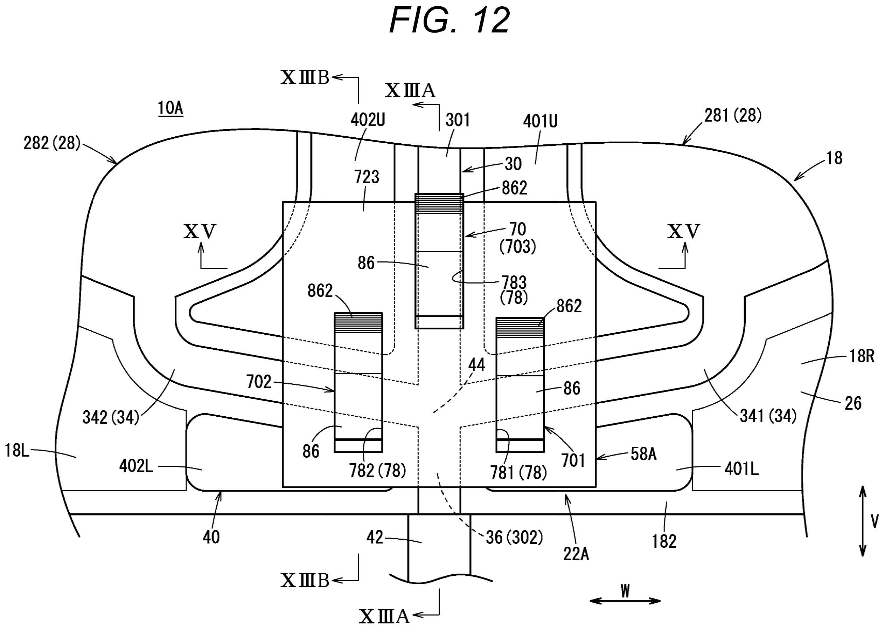

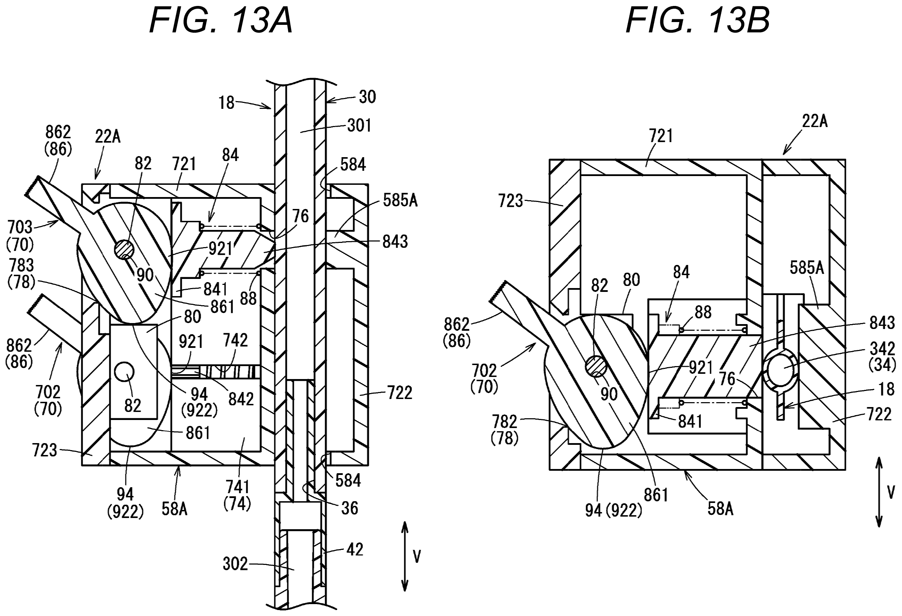

As illustrated in Fig. 12, the channel switching portion 22A includes single housing 58A and a plurality of blocking portions 70. As illustrated in Fig. 13A, the housing 58A movably holds a blocking member 84. The housing 58A includes a body main body 721, a bottom portion 722 provided at a lower portion of the body main body 721 and constituting a bottom, and a cover portion 723 covering an upper portion of the body main body 721.

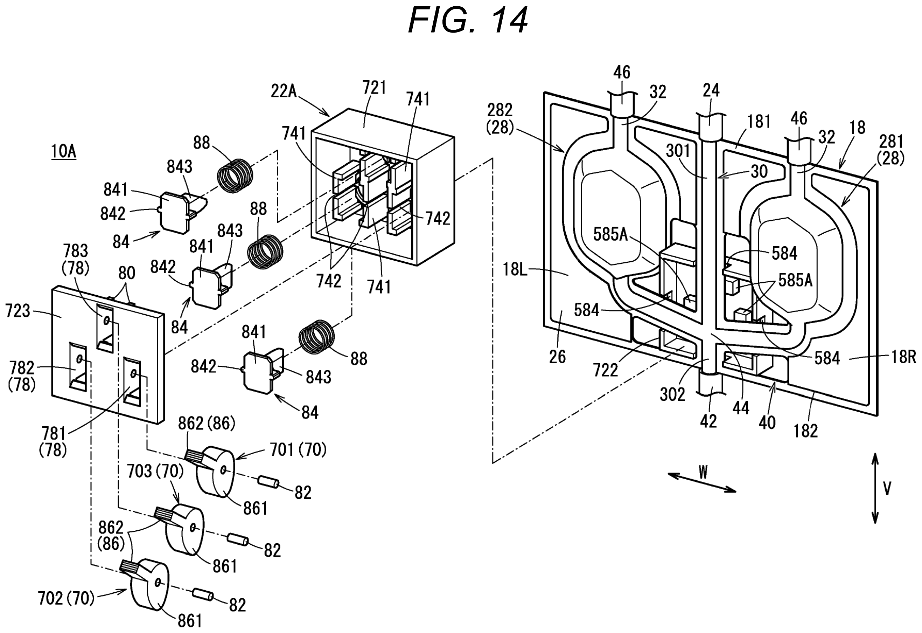

As illustrated in Fig. 14, the body main body 721 is formed in a box shape. The body main body 721 includes a plurality of guide portions 74 therein. Each of the guide portions 74 has a set of guide walls 741 extending in the up-down direction of the body main body 721. The set of guide walls 741 are opposed to and parallel to each other. Each of the guide walls 741 includes a guide groove 742. The guide groove 742 extends in the up-down direction. As illustrated in Fig. 13A, the bottom of the body main body 721 has a hole portion 76 penetrating the bottom.

The bottom portion 722 covers the bottom of the body main body 721. A part of the collection bag 18 is accommodated within the bottom portion 722. As illustrated in Fig. 14, the connection portion 44 of the collection bag 18 is accommodated within the bottom portion 722. The inflow-side channel 301 and the outflow-side channel 302 of the main channel 30, and the first connection channel 341 and the second connection channel 342 are disposed in each recessed portion 584 of the side wall of the bottom portion 722. The bottom wall of the bottom portion 722 has a plurality of pedestals 585A. The plurality of pedestals 585A are disposed at positions facing the inflow-side channel 301 of the main channel 30, the first connection channel 341, and the second connection channel 342. The plurality of pedestals 585A face the hole portion 76 of the body main body 721.

The cover portion 723 covers the upper portion of the body main body 721. As illustrated in Fig. 13A, the cover portion 723 includes a plurality of openings 78 and a plurality of support portions 80 provided within the cover portion 723. The plurality of openings 78 penetrate the cover portion 723. Each set of the plurality of support portions 80 is disposed outward in the width direction of each of the openings 78. Each of the support portions 80 protrudes in a direction orthogonal to the cover portion 723. A pin member 82 that supports each of the blocking portions 70 to be described later is supported by each set of support portions 80.

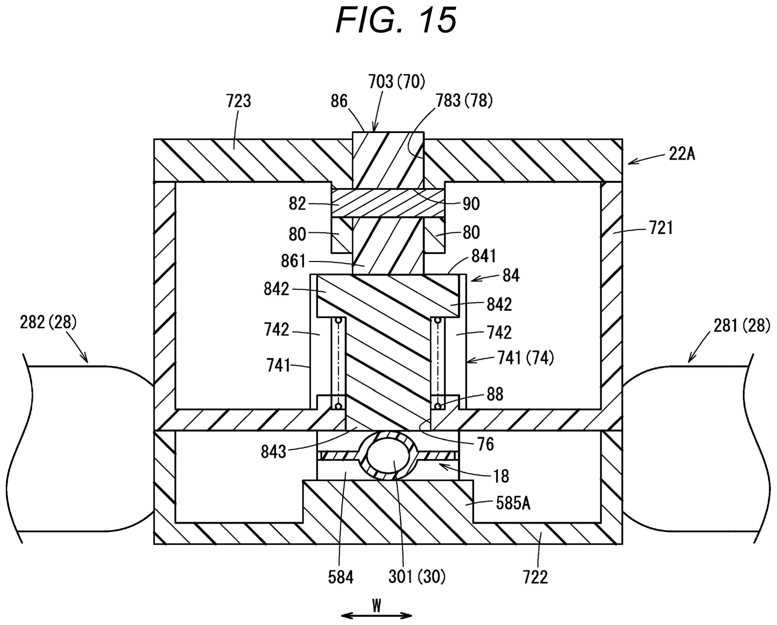

The pin member 82 faces the opening 78 and extends in a direction intersecting the opening direction of the opening 78 (see Fig. 15). As illustrated in Fig. 12, the plurality of openings 78 respectively include a first opening 781, a second opening 782, and a third opening 783. The first opening 781 is disposed on one side in the width direction of the cover portion 723 and faces the first connection channel 341 of the collection bag 18. The second opening 782 is disposed in parallel with the first opening 781 and disposed on the other side in the width direction of the cover portion 723. The second opening 782 faces the second connection channel 342 of the collection bag 18. The third opening 783 is disposed between the first opening 781 and the second opening 782 and faces the inflow-side channel 301.

The plurality of blocking portions 70 can block the first connection channel 341 and the second connection channel 342, respectively. The plurality of blocking portions 70 include a first blocking portion 701 capable of blocking the first connection channel 341 and a second blocking portion 702 capable of blocking the second connection channel 342. The first blocking portion 701 is disposed in the first opening 781 of the cover portion 723. The second blocking portion 702 is disposed in the second opening 782 of the cover portion 723. Each of the blocking portions 70 further includes a third blocking portion 703 capable of blocking the inflow-side channel 301 of the main channel 30. The third blocking portion 703 is disposed in the third opening 783 of the cover portion 723.

In the present embodiment, since the first blocking portion 701, the second blocking portion 702, and the third blocking portion 703 are similarly configured, the third blocking portion 703 will be described in detail below. The first blocking portion 701 and the second blocking portion 702 are the same as the third blocking portion 703 except in that they are respectively mounted on the first opening 781 and the second opening 782.

As illustrated in Fig. 13A, the third blocking portion 703 includes a blocking member 84 and a pressing member 86. The blocking member 84 is provided to be movable in the thickness direction of the collection bag 18. As illustrated in Fig. 15, the blocking member 84 is disposed between the set of guide walls 741 of the body main body 721. When the blocking member 84 moves, the blocking member 84 is guided in the thickness direction of the collection bag 18 by the set of guide portions 74. The proximal end of the blocking member 84 is disposed on the side of the cover portion 723 and has a pressed portion 841. The pressed portion 841 has a surface orthogonal to the axial direction of the blocking member 84. The proximal end of the blocking member 84 has a protrusion 842 protruding laterally from the pressed portion 841, and the protrusion 842 is inserted into the guide groove 742 of the guide wall 741.

As illustrated in Fig. 13A, the distal end of the blocking member 84 has a blocking portion 843. The blocking portion 843 is tapered toward the distal end. The blocking portion 843 is inserted into the hole portion 76 of the body main body 721. When the blocking member 84 moves toward the collection bag 18, the blocking portion 843 presses and blocks the inflow-side channel 301 of the main channel 30.

An elastic member 88 is provided on an outer periphery of the blocking member 84. The elastic member 88 is, for example, a coil spring. The elastic member 88 is provided between the protrusion 842 of the blocking member 84 and the bottom of the body main body 721. The elastic member 88 has an elastic force that biases the blocking member 84 in a direction moving away from the collection bag 18

The pressing member 86 is provided in the housing 58A. The pressing member 86 faces the pressed portion 841 of the blocking member 84. The pressing member 86 includes a cam portion 861 and an operation portion 862. The cam portion 861 has a pin hole 90 into which the pin member 82 is inserted. The cam portion 861 includes a first pressing portion 921 having an outer peripheral surface with a constant diameter centered on the pin hole 90, and a second pressing portion 922 having a larger radius centered on the pin hole 90 than the outer peripheral surface of the first pressing portion 921. The second pressing portion 922 has a top portion 94 most spaced apart from the pin hole 90. The outer peripheral surface of the cam portion 861 comes into contact with the pressed portion 841 of the blocking member 84. The pin member 82 inserted through the support portion 80 of the cover portion 723 is inserted into the pin hole 90 of the cam portion 861. The pressing member 86 including the cam portion 861 is rotatably supported by the pin member 82.

The operation portion 862 is exposed to the outside of the housing 58A through the opening 78. The operation portion 862 is gripped by the user when the opened/blocked state of the inflow-side channel 301 is switched by the third blocking portion 703. The operation portion 862 protrudes radially outward from the outer peripheral surface of the first pressing portion 921. The operation portion 862 is disposed on the side opposite to the top portion 94 of the second pressing portion 922 with the pin hole 90 as a center.

The operation portion 862 is inserted into the opening 78 and exposed to the outside of the cover portion 723. The pressing member 86 is rotatably supported relative to the set of support portions 80.

As illustrated in Fig. 13A, when the first pressing portion 921 of the cam portion 861 is in contact with the pressed portion 841 of the blocking member 84, the blocking member 84 is not pressed toward the collection bag 18, and the blocking portion 843 of the blocking member 84 is spaced apart from the inflow-side channel 301, resulting in the open position. At this time, the operation portion 862 is disposed so as to protrude upward.

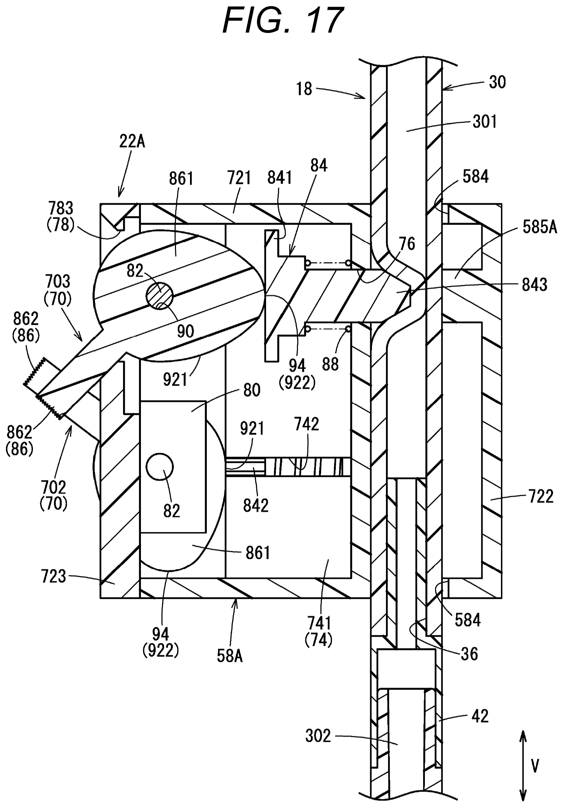

When the user operates the operation portion 862 of the pressing member 86, the pressing member 86 presses the blocking member 84 toward the inflow-side channel 301. As illustrated in Fig. 17, when the operation portion 862 is operated downward to rotate the pressing member 86 and the second pressing portion 922 of the cam portion 861 is in contact with the pressed portion 841 of the blocking member 84, the blocking member 84 is pressed toward the collection bag 18 by the top portion 94 of the cam portion 861. Since the radius of the second pressing portion 922 relative to the pin hole 90 is greater than the radius of the first pressing portion 921 relative to the pin hole 90, the second pressing portion 922 causes the blocking member 84 to move toward the collection bag 18 while compressing the elastic member 88. The inflow-side channel 301 is pressed toward the pedestal 585A and blocked by the blocking portion 843 of the blocking member 84. At this time, the operation portion 862 is disposed so as to protrude downward.

The blocking member 84 of the first blocking portion 701 is provided so as to be movable toward the first connection channel 341. The pressing member 86 of the first blocking portion 701 is provided so as to be exposed to the outside from the first opening 781. When the user operates the operation portion 862 of the pressing member 86 of the first blocking portion 701 downward, the pressing member 86 pushes the blocking member 84 toward the first connection channel 341.

As illustrated in Fig. 13B, the blocking member 84 of the second blocking portion 702 is provided so as to be movable toward the second connection channel 342. The pressing member 86 of the second blocking portion 702 is provided so as to be exposed to the outside from the second opening 782. When the user operates the operation portion 862 of the pressing member 86 of the second blocking portion 702 downward, the pressing member 86 pushes the blocking member 84 toward the second connection channel 342. The first connection channel 341 and the second connection channel 342 can be selectively pressed by the blocking members 84 of the first blocking portion 701 and the second blocking portion 702. The blocking member 84 of the third blocking portion 703 is provided so as to be movable toward the inflow-side channel 301 of the main channel 30. The third blocking portion 703 can selectively press the inflow-side channel 301 of the main channel 30.

Note that the present invention is not limited to the case where the plurality of blocking portions 70 are provided in the single housing 58A. For example, a plurality of housings holding each of the plurality of blocking portions 70 (the first blocking portion 701, the second blocking portion 702, the third blocking portion 703) may be provided, and the housings may be mounted on the first connection channel 341, the second connection channel 342, and the inflow-side channel 301.

Next, the operation of the channel switching portion 22A in a case where the collection target M is dispensed into the first sampling container 141 and the second sampling container 142 using the collection kit 10A according to the second embodiment will be described.

First, in the connection step of connecting the medical bag 12 to the inflow tube 16, as illustrated in Fig. 12, in the channel switching portion 22A, the operation portion 862 of the pressing member 86 of each of the first blocking portion 701, the second blocking portion 702, and the third blocking portion 703 is disposed to face upward of the collection bag 18, resulting in the open position. A state where all of the first blocking portion 701, the second blocking portion 702, and the third blocking portion 703 illustrated in Fig. 12 are in the open position will be described as the initial position. At the initial position, the first connection channel 341, the second connection channel 342, and the inflow-side channel 301 communicate with each other via the connection portion 44.

A step of introducing the collection target M from the medical bag 12 to each of the first accommodation chamber 281 and the second accommodation chamber 282 through the inflow tube 16, the inflow-side channel 301, the first connection channel 341, and the second connection channel 342, and then transferring the collection target M in the first accommodation chamber 281 to the first sampling container 141 is performed.

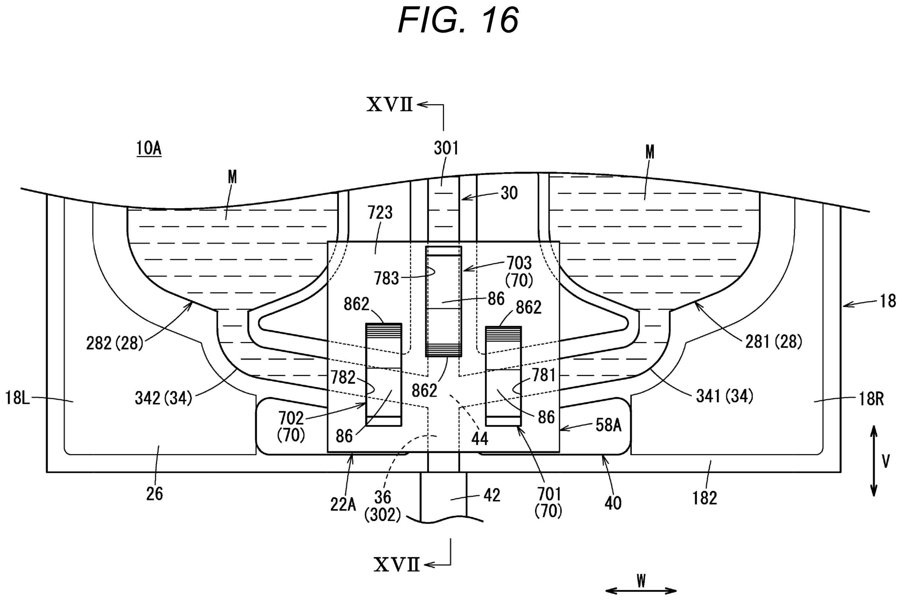

As illustrated in Fig. 16, the inflow-side channel 301 is blocked by the third blocking portion 703 of the channel switching portion 22A. The user operates the operation portion 862 of the third blocking portion 703 to rotate the pressing member 86 such that the operation portion 862 moves downward to reach the blocking position as illustrated in Fig. 17. The second pressing portion 922 faces the pressed portion 841 of the blocking member 84 by the rotation of the pressing member 86, and the blocking member 84 is pressed toward the collection bag 18 by the second pressing portion 922. The blocking member 84 moves toward the inflow-side channel 301 while compressing the elastic member 88, and the blocking portion 843 of the blocking member 84 presses the inflow-side channel 301 toward the pedestal 585A. The inflow-side channel 301 is blocked by the blocking member 84. The pressing member 86 of the third blocking portion 703 is held in a state of pressing the blocking member 84.

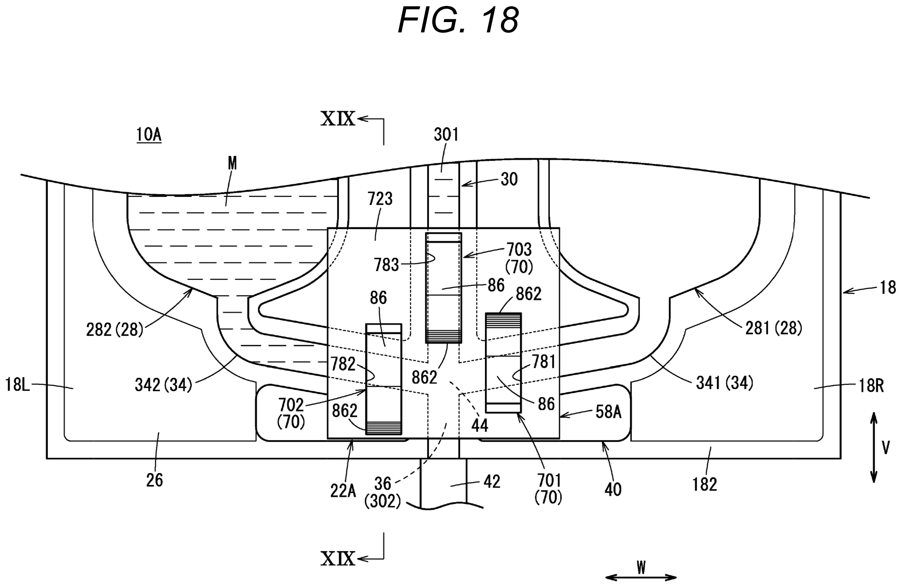

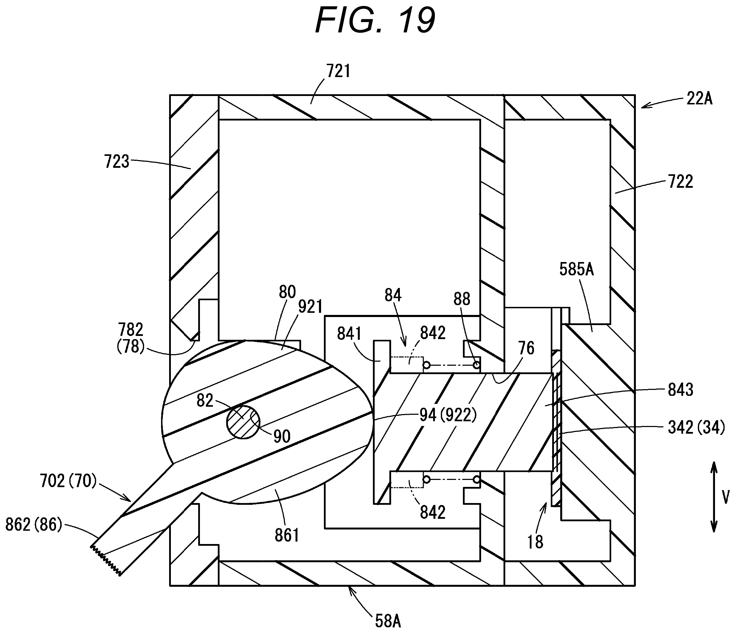

As illustrated in Fig. 18, the second connection channel 342 is blocked by the second blocking portion 702 of the channel switching portion 22A. The user operates the operation portion 862 of the second blocking portion 702 downward, and rotates the pressing member 86 by the operation of the operation portion 862, resulting in the blocking position, as illustrated in Fig. 19. The second pressing portion 922 faces the pressed portion 841 of the blocking member 84 by the rotation of the pressing member 86, and the blocking member 84 is pressed toward the collection bag 18 by the second pressing portion 922. The blocking member 84 moves toward the second connection channel 342 while compressing the elastic member 88, and the blocking portion 843 of the blocking member 84 presses the second connection channel 342 toward the pedestal 585A. The second connection channel 342 is blocked by the blocking member 84. The pressing member 86 of the second blocking portion 702 is held in a state of pressing the blocking member 84.

By connecting the first sampling container 141 to the adapter 20, the collection target M in the first accommodation chamber 281 is transferred to the first sampling container 141 through the first connection channel 341, the outflow-side channel 302, and the outflow port 36.

Next, after the first sampling container 141 is removed from the adapter 20, a step of transferring the collection target M in the second accommodation chamber 282 to the second sampling container 142 is performed.

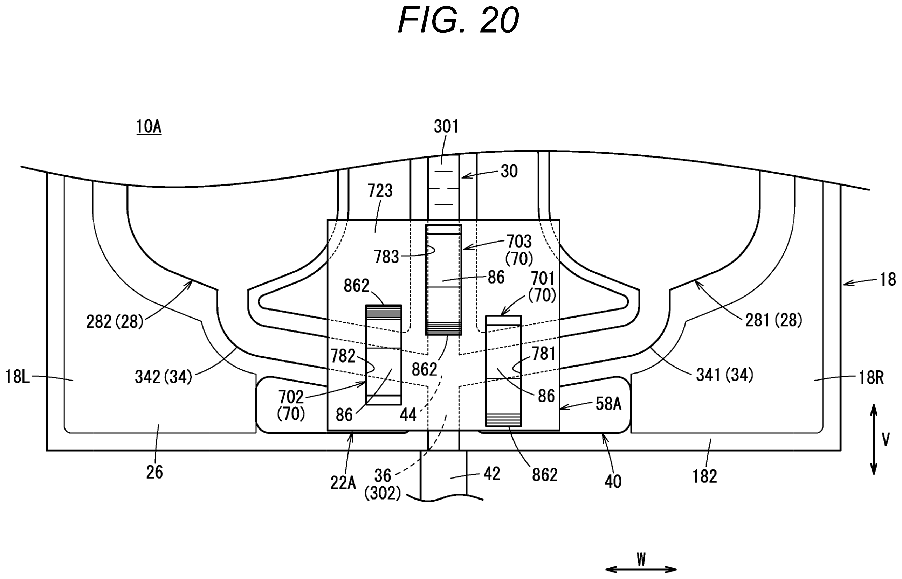

After the second sampling container 142 is connected to the adapter 20, the first connection channel 341 is blocked by the first blocking portion 701 of the channel switching portion 22A as illustrated in Fig. 20. The user operates the operation portion 862 of the first blocking portion 701 to rotate the pressing member 86 such that the operation portion 862 moves downward to reach the blocking position. By the rotation of the pressing member 86, the blocking member 84 moves toward the first connection channel 341, and the first connection channel 341 is blocked by the blocking portion 843 of the blocking member 84. The pressing member 86 of the first blocking portion 701 is held in a state of pressing the blocking member 84.

The second connection channel 342 is opened by the second blocking portion 702 of the channel switching portion 22A. The user operates the operation portion 862 of the second blocking portion 702 to rotate the pressing member 86 such that the operation portion 862 moves upward to reach the open position. Since the first pressing portion 921 faces the pressed portion 841 of the blocking member 84 by the rotation of the pressing member 86, the pressing force applied to the blocking member 84 by the pressing member 86 decreases, and as illustrated in Fig. 13B, the blocking member 84 moves in a direction away from the collection bag 18 due to the elastic force of the elastic member 88. The blocking portion 843 of the blocking member 84 is separated from the second connection channel 342, and the second connection channel 342 returns to its original shape due to elasticity. Blocking of the second connection channel 342 is released, and thus the second accommodation chamber 282 and the outflow-side channel 302 communicate with each other through the second connection channel 342.

The collection target M in the second accommodation chamber 282 is transferred to the second sampling container 142 through the second connection channel 342, the outflow-side channel 302, and the outflow port 36.

Note that, the case where the blocking member 84 of the third blocking portion 703 is pushed toward the inflow-side channel 301 to block the inflow-side channel 301 when the collection target M is transferred from the first accommodation chamber 281 and the second accommodation chamber 282 to the first sampling container 141 and the second sampling container 142 has been described, but the collection target M may be transferred in a state where the inflow-side channel 301 is opened.

According to the second embodiment, as illustrated in Fig. 12, the channel switching portion 22A includes the plurality of blocking portions 70 (the first blocking portion 701, and the second blocking portion 702) capable of respectively blocking the plurality of connection channels 34 (the first connection channel 341, and the second connection channel 342). The plurality of blocking portions 70 include a plurality of blocking members 84 that are respectively movable toward the plurality of connection channels 34, and the plurality of blocking members 84 can selectively press the plurality of connection channels 34.

Thus, by moving each of the blocking members 84 of the plurality of blocking portions 70, it is possible to selectively press and block the plurality of connection channels 34.

The channel switching portion 22A includes a single housing 58A that movably holds the plurality of blocking members 84, and the plurality of blocking portions 70 respectively include a plurality of operation portions 862 provided in the housing 58A and exposed from the housing 58A. As illustrated in Fig. 14, a plurality of pressing members 86 are provided to push the blocking member 84 toward each of the plurality of connection channels 34 when the user operates the operation portion 862.

Thus, by operating each of the plurality of operation portions 862, the blocking member 84 can be effectively moved toward each of the plurality of connection channels 34 by the pressing member 86. By providing the plurality of blocking portions 70 in the single housing 58A, the channel switching portion 22A can be compactly configured.

Note that, the present invention is not limited to the above disclosure and can take various configurations without departing from the gist of the present invention.

## Claims

1. A collection kit for collecting a collection target accommodated in a medical bag into a plurality of sampling containers, the collection kit comprising:
an inflow tube to which the medical bag is connected; and
a collection bag that is connected to a downstream side of the inflow tube and is formed by overlapping and welding two flexible sheets,
wherein the collection bag includes
a plurality of accommodation chambers that accommodate the collection target,
a single outflow port for transferring the collection target from a plurality of the accommodation chambers to a plurality of the sampling containers, respectively,
at least one exhaust portion for exhausting air within a plurality of the accommodation chambers,
a single main channel that connects the inflow tube and the outflow port, and
a plurality of connection channels that are provided between the main channel and a plurality of the accommodation chambers and connect each of the accommodation chambers and the main channel.

2. The collection kit according to claim 1,
wherein the collection bag includes a connection portion to which a plurality of the connection channels and the main channel are connected,
the main channel has an inflow-side channel connecting the connection portion and the inflow tube, and an outflow-side channel connecting the connection portion and the outflow port, and
the collection kit includes a channel switching portion that selectively connects any one of a plurality of the connection channels and the outflow-side channel.

3. The collection kit according to claim 2,
wherein the collection bag includes a cutout portion obtained by cutting out a part of the flexible sheets in a thickness direction of the collection bag so as to surround the connection portion, and
the channel switching portion is provided in the cutout portion.

4. The collection kit according to claim 2 or 3,
wherein the channel switching portion includes a switching member rotatable relative to the collection bag,
the switching member includes a blocking portion that selectively blocks any one of a plurality of the connection channels, and
one of a plurality of the connection channels is capable of being blocked by the blocking portion by selecting a rotational position of the switching member.

5. The collection kit according to claim 4,
wherein the channel switching portion includes a housing that rotatably holds the switching member,
the switching member is provided so as to be movable in a thickness direction of the collection bag relative to the housing, and the switching member moves toward the connection channel in the thickness direction, thereby causing the blocking portion to press the connection channel toward the housing.

6. The collection kit according to claim 4,
wherein the blocking portion of the switching member is capable of blocking the inflow-side channel, and
one of a plurality of the connection channels and the inflow-side channel are capable of being blocked by the blocking portion by selecting the rotational position of the switching member.

7. The collection kit according to claim 2 or 3,
wherein the channel switching portion includes a plurality of blocking portions capable of respectively blocking a plurality of the connection channels,
a plurality of the blocking portions include a plurality of blocking members respectively movable toward a plurality of the connection channels, and
a plurality of the blocking members are capable of selectively pressing a plurality of the connection channels.

8. The collection kit according to claim 7,
wherein the channel switching portion includes a single housing that movably holds a plurality of the blocking members, and
a plurality of the blocking portions include a plurality of pressing members respectively having a plurality of operation portions provided in the housing and exposed from the housing, the pressing members pushing the blocking members toward each of a plurality of the connection channels by operating the operation portions.

9. A collection method for collecting a collection target accommodated in a medical bag into a plurality of sampling containers via a collection kit,
wherein the collection kit includes
an inflow tube to which the medical bag is connected, and
a collection bag that is connected to a downstream side of the inflow tube and is formed by overlapping and welding two flexible sheets,
the collection bag includes
a plurality of accommodation chambers that accommodate the collection target,
a single outflow port for transferring the collection target from a plurality of the accommodation chambers to a plurality of the sampling containers, respectively,
at least one exhaust portion for exhausting air within a plurality of the accommodation chambers,
a single main channel that connects the inflow tube and the outflow port,
a plurality of connection channels that are provided between the main channel and a plurality of the accommodation chambers, and connect each of the accommodation chambers and the main channel,
a connection portion that connects a plurality of the connection channels and the main channel,
an inflow-side channel that is provided in the main channel and connects the connection portion and the inflow tube, and
an outflow-side channel that is provided in the main channel and connects the connection portion and the outflow port,
the collection method comprises:
a connection step of connecting the medical bag to the inflow tube;
an introduction step of introducing the collection target having moved from the medical bag to an inside of the collection bag through the inflow tube and the inflow-side channel into each of a plurality of the accommodation chambers through a plurality of the connection channels;
a separation step of separating the medical bag from the inflow tube after the collection target is introduced into each of a plurality of the accommodation chambers; and
a collection step of sequentially transferring the collection target from a plurality of the accommodation chambers to a plurality of the sampling containers by sequentially connecting a plurality of the sampling containers to the outflow port and selectively blocking and opening a plurality of the connection channels.
